# EUROPÄISCHE PATENTSCHRIFT

(11) **EP 0 427 160 B1**
(45) Veröffentlichungstag und Bekanntmachung des Hinweises auf die Patenterteilung: **05.04.1995**
(21) Anmeldenummer: 90121096.3
(22) Anmeldetag: 03.11.1990
(51) Int. Cl.: C07D 213/75, C07D 213/40, A61K 31/44

(54) **Benzylselenobenzamide von Aminopyridinen und Picolylaminen**
Benzylselenobenzamides of aminopyridines and picolylamines
Benzylsélénobenzamides d'aminopyridines et de picolylamines

(30) Priorität: 08.11.1989 DE 3937170
(43) Veröffentlichungstag der Anmeldung: 15.05.1991
(73) Patentinhaber: A. Nattermann & Cie. GmbH, D-50829 Köln (DE)
(72) Erfinder: Biedermann, Jürgen, Dr., W-5024 Pulheim 3 (DE); Evers, Michel, Dr., B-4000 Liege (BE); Terlinden, Rolf, Dr., W-5000 Köln 71 (DE); Leyck, Sigurd, Dr., W-5024 Pulheim 2 (DE); Graf, Erich, Dr., W-5014 Kerpen-Horrem (DE)
(74) Vertreter: Döring, Wolfgang, Dr.-Ing. Patentanwälte Hauck, Graalfs, Wehnert, Döring, Siemons

(56) Entgegenhaltungen:
- DE-A- 3 443 467
- DE-A- 3 626 554
- Arzneimittelchemie I, Georg Thieme Verlag, Stuttgart, DE, 1976 ; Schröder et al : "Grundlagen Nerven, Muskeln und Gewebe", Seiten 32,33
- Multidimensional Pharmacochemistry, Academic Press, Inc., 1984 ; Peter P. Mager :"Design of safer drugs", p. 33
- Burger's Medicinal Chemistry, 4th Edition, Part 1, The Basis of Medicinal Chemistry, A Wiley-Interscience Publication, 1979 , p. 6
- Medical Chemistry, 3rd Edition, Part 1, Wiley-Interscience, 1980, pp. 72,73,75 to 77

## Beschreibung

Die Erfindung betrifft neue Benzylselenobenzamide, Verfahren und Zwischenprodukte zu ihrer Herstellung und diese Verbindungen enthaltende pharmazeutische Präparate.

Die erfindungsgemäßen Verbindungen entsprechen der allgemeinen Formel I 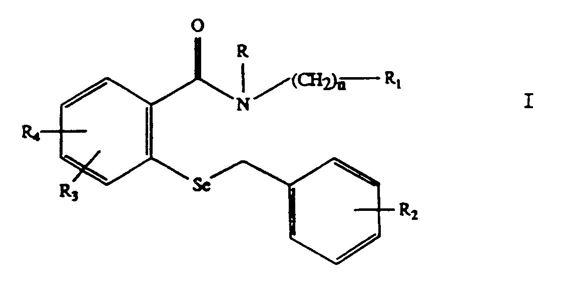
worin
- R: Wasserstoff, Methyl oder Ethyl bedeutet und
- R₁: für die Pyridylgruppe steht, die gegebenenfalls durch Fluor, Chlor, Brom, C₁₋₄-Alkyl, C₁₋₄-Alkoxy, Hydroxy, Cyano, Amino, Dimethylamino oder Nitro substituiert ist und
- R₂,R₃ und R₄: gleich oder verschieden sind und unabhängig voneinander für Wasserstoff, Fluor, Chlor, Brom, Trifluormethyl, C₁₋₄-Alkyl, C₁₋₄-Alkoxy, Hydroxy, Cyano oder Nitro stehen oder
- R₃ ,R₄: zusammen für Methylendioxy stehen und
- n: Null oder 1 bedeutet.

Besonders bevorzugt sind dabei die Verbindungen der allgemeinen Formel I, in denen n gleich Null ist und R und R₂ Wasserstoff bedeuten, während R₁ für die Pyridylgruppe steht und R₃ und R₄ gleich oder verschieden sind und unabhängig voneinander Wasserstoff, Fluor, Chlor, Brom, Trifluormethyl, Methyl, Methoxy, Hydroxy, Cyano oder Nitro bedeuten oder R₃ und R₄ zusammen für Methylendioxy stehen. Ebenfalls bevorzugt sind die Verbindungen der allgemeinen Formel I, worin n gleich 1 ist und R und R₂ Wasserstoff bedeuten, R₁ einen unsubstituierten Pyridylrest darstellt und R₃ und R₄ gleich oder verschieden sind und unabhängig voneinander Wasserstoff, Fluor, Chlor, Brom, Trifluormethyl, Methyl, Methoxy, Hydroxy, Cyano oder Nitro
bedeuten oder R₃ und R₄ zusammen für Methylendioxy stehen.

Erfindungsgemäße Verbindungen sind beispielsweise: 2-Benzylseleno-N-(2-pyridyl)benzamid
2-Benzylseleno-N-(3-pyridyl)benzamid
2-Benzylseleno-N-(4-pyridyl)benzamid
2-Benzylseleno-N-(2-picolyl)benzamid
2-Benzylseleno-N-(3-picolyl)benzamid
2-Benzylseleno-N-(3-pyridyl)-3-trifluormethylbenzamid
2-Benzylseleno-N-(4-picolyl)benzamid
2-Benzylseleno-3-fluor-N-(3-pyridyl)benzamid
Die erfindungsgemäßen Verbindungen können entweder aus den entsprechenden 2-Benzylselenobenzoesäuren der allgemeinen Formel II 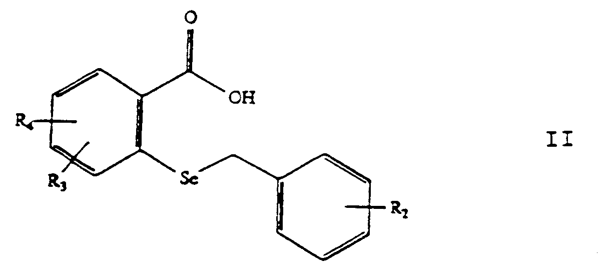
durch Umsetzung mit Chlormethylen-dimethyliminiumchlorid (Vilsmeier-Reagenz)-nach Reaktionschema 1- und der umzusetzenden Pyridyl- oder Picolylverbindung in einem chlorierten Kohlenwasserstoff erhalten werden, oder aber die Säure wird als Imidazolid (nach Reaktionschema 2) eingesetzt und mit Pyridylamin oder Picolylamin umgesetzt.

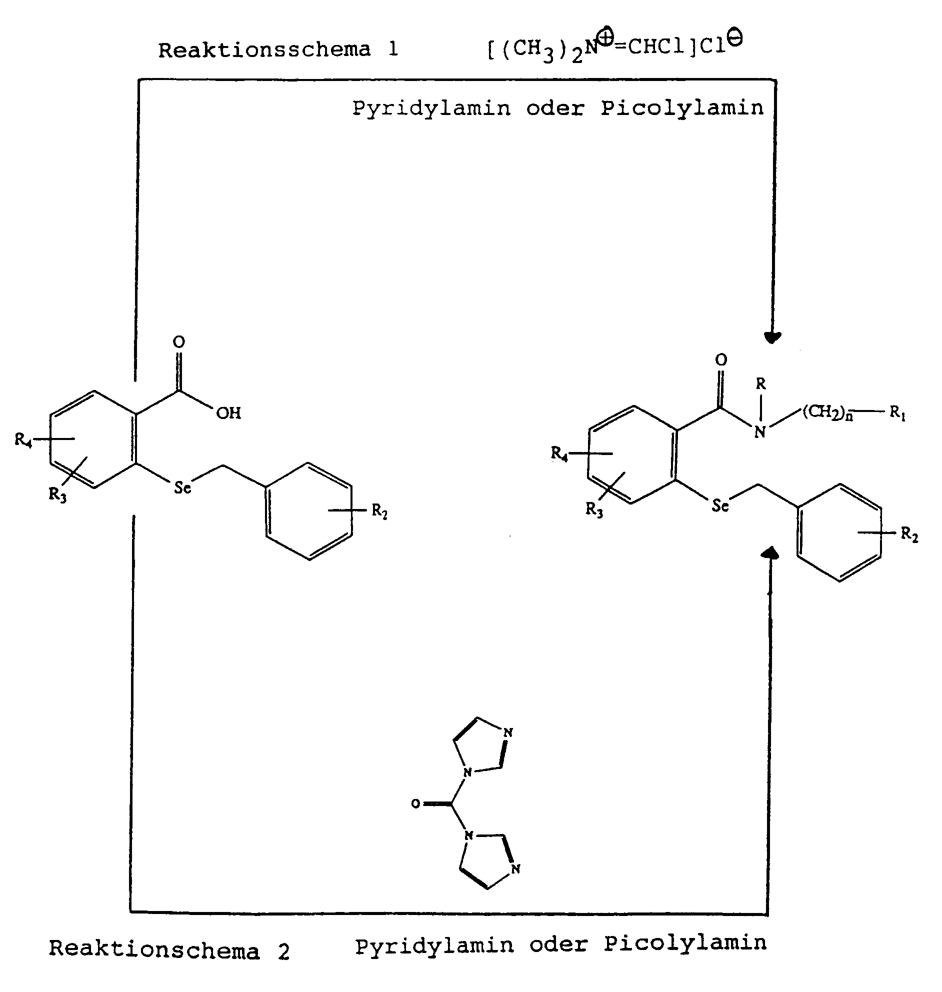

Ein anderer Weg führt über die Umsetzung einer Verbindung der Formel II mit 2-Chlor-1-methylpyridiniumjodid (Reaktionschema 3) und einem Pyridin-haltigen Amin.

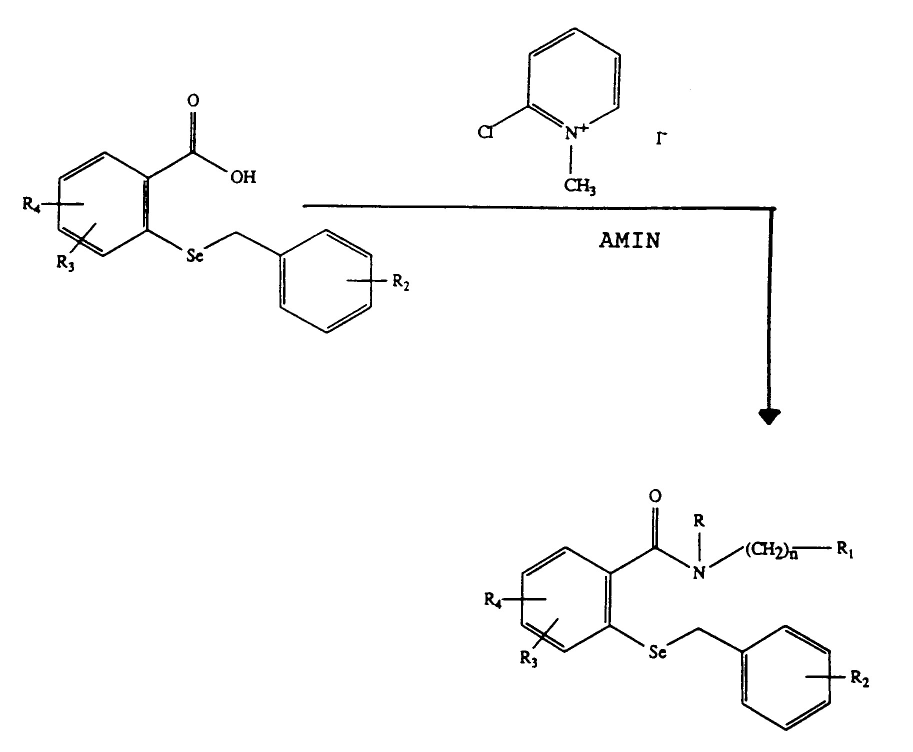

Als Ausgangsprodukte für die Synthese der erfindungsgemäßen Verbindungen eignen sich beispielsweise:
2-Benzylselenobenzoesäure
2-(4-Methylbenzylseleno)benzoesäure
2-(4-Methoxybenzylseleno)benzoesäure
2-(4-Brombenzylseleno)benzoesäure
2-(4-Cyanobenzylseleno)benzoesäure
2-(4-Nitrobenzylseleno)benzoesäure
2-(4-Fluorbenzylseleno)benzoesäure
2-(4-Chlorbenzylseleno)benzoesäure
2-Benzylseleno-3-methoxy-benzoesäure
2-Benzylseleno-3-fluor-benzoesäure
2-Benzylseleno-3,4-methylendioxybenzoesäure
2-Benzylseleno-3-trifluormethyl-benzoesäure
2-Benzylseleno-4-methyl-benzoesäure

Die vorliegende Erfindung betrifft ebenfalls pharmazeutische Präparate, welche Verbindungen der Formel I als Wirkstoffe enthalten. Bei den erfindungsgemäßen pharmazeutischen Präparaten handelt es sich um solche zur enteralen wie oralen oder rektalen sowie parenteralen Verabreichung, welche die pharmazeutischen Wirkstoffe allein oder zusammen mit einem üblichen, pharmazeutisch anwendbaren Trägermaterial enthalten. Vorteilhafterweise liegt die pharmazeutische Zubereitung des Wirkstoffes in Form von Einzeldosen vor, die auf die gewünschte Verabreichung abgestimmt sind, wie z.B. Tabletten, Dragées, Kapseln, Suppositorien, Granulate, Lösungen, Emulsionen oder Suspensionen. Die Dosierung der Substanzen liegt üblicherweise zwischen 10 und 1000 mg pro Tag, vorzugsweise zwischen 30 und 300 mg pro Tag, und kann in einer Dosis oder mehreren Teildosen, vorzugsweise in zwei bis drei Teildosen pro Tag, verabreicht werden.

Es konnte Festgestellt werden, daß ein derartiges pharmazeutisches Präparat hervorragende entzündungshemmende Eigenschaften besitzt.

Die Herstellung der erfindungsgemäßen Verbindungen wird durch die folgenden Beispiele näher erläutert.

Die angegebenen Schmelzpunkte wurden mit einem Büchi 510- Schmelzpunktbestimmungsapparat gemessen und sind mit °C angegeben und nicht korrigiert.

### Beispiel 1

### 2-Benzylselenobenzoesäure

Zu einer Suspension von 50,0 g (0,125 Mol) Diselenosalicylsäure in 380 ml Wasser werden unter Rühren 38,0 g (0,95 Mol) Natriumhydroxid gegeben, wobei die Innentemperatur auf 45°C ansteigt und die Säure in Lösung geht. Nach Zugabe von 100 g (0,94 Mol) Natriumcarbonat und 58 g (0,333 Mol) Natriumdithionit steigt die Temperatur auf 55°C. Man erhitzt 2 Stunden unter Rückfluß, läßt abkühlen, und tropft bei Raumtemperatur innerhalb 10 Minuten
47,6 ml (68,4 g; 0,4 Mol) Benzylbromid zu. Nach weiterer Wasserzugabe (100 ml) wird 14 Stunden bei Raumtemperatur weitergerührt, dann werden 355 ml Salzsäure (32 %) unter Rühren zum Reaktionsgemisch gegeben, die ausgefallene weiße Festsubstanz abgesaugt und mit Wasser neutral gewaschen.

Das noch feuchte Rohprodukt wird unter Zusatz von 3g Aktiv-Kohle aus
1600 ml 2-Propanol umkristallisiert. Die Ausbeute beträgt 48,8 g (67% d. Th.). Aus der Mutterlauge werden weitere 12 g (16,5 %) 2-Benzylselenobenzoesäure gewonnen.
Ausbeute: 60,8 g (83,5% d. Th.)
Fp.: 209-210°C

### Beispiel 2

### 2-Benzylseleno-N-(2-pyridyl)-benzamid

2,94 g (0,0086 Mol) 2-Benzylselenobenzoesäure-imidazplid (erhalten aus 2-Benzylselenobenzoesäure gemäß Beispiel 1 und N,N'-Carbonyldiimidazol in Tetrahydrofuran) und 0,81 g (0,0086 Mol) 2-Aminopyridin werden in 20 ml Dimethylformamid gelöst und 14 Stunden auf 150°C erhitzt. Dann werden 200 ml Wasser zugesetzt und das ölige Reaktionsprodukt mit zweimal je 50 ml Dichlormethan extrahiert. Die organische Phase wird getrocknet und eingeengt und nacheinander aus 2-Propanol (20 ml) und Toluol (20 ml) umkristallisiert. Ein Restbestand an 2-Benzylselenobenzoesäure wird durch Zermahlen der Kristalle, Rühren mit 1 N NaOH (50 ml), Neutral-Waschen und Umkristallisation aus 2-Propanol (20 ml) entfernt.
Ausbeute: 0,65 g (20,6% d.Th.)
Fp.: 134 - 135 °C

### Beispiel 3

### 2-Benzylseleno-N-(3-pryidyl)-benzamid

In eine auf -5°C abgekühlte Suspension von 5,82 g (0,02 Mol) 2-Benzylselenobenzoesäure aus Beispiel 1 in 30 ml Dichlormethan werden unter Rühren 2,82 g (0,022 Mol) Chlormethylen-dimethyliminiumchlorid (Vilsmeier-Reagenz) eingetragen und 2 Stunden bei Raumtemperatur gerührt. Dann werden innerhalb von 10 Minuten eine Lösung von 2,07 g (0,022 Mol) 3-Aminopyridin und 4,79 g (0,047 Mol) Triethylamin in 30 ml Dichlormethan zugetropft und 14 Stunden gerührt, anschließend mit 50 ml Wasser ausgeschüttelt. Der weder in Wasser noch in Dichlormethan lösliche Feststoff wird abgesaugt (Hydrochlorid des Reaktionsprodukts) und in eine Mischung aus 60 ml Essigsäureethylester, 40 ml Diethylether und 2 ml Ethanol suspendiert und mit 50 ml 1 N NaOH gerührt. Die organische Phase wird abgetrennt, mit Wasser neutral gewaschen, getrocknet und eingeengt und der Rückstand wird aus 2-Propanol (60 ml) umkristallisiert.
Ausbeute: 3,0 g (40,8% d.Th.)
Fp.: 151 - 153°C

### Beispiel 4

### 2-Benzylseleno-N-(4-pyridyl)benzamid

Zu einer Lösung von 2,91 g (0,01 Mol) 2-Benzylselenobenzoesäure (aus Beispiel 1) und 1,4 ml (0,01 Mol) Triethylamin in 25 ml Dichlormethan gibt man 2,56 g (0,01 Mol) 2-Chlor-1-methylpyridiniumjodid und rührt 1 Stunde.
Dann gibt man eine Lösung von 0,94 (0,01 Mol) 4-Aminopyridin und 1,4 ml (0,01 Mol) Triethylamin in 25 ml Dichlormethan zu und rührt 2 Stunden. Anschließend wird zweimal mit je 50 ml Wasser ausgeschüttelt, getrocknet und eingeengt. Zuletzt wird aus 2-Propanol (40 ml) / n-Hexan (40 ml) umkristallisiert.
Ausbeute : 1,8 g (49% d. Th.)
Fp.: 58° C

### Beispiel 5

### 2-Benzylseleno-N-(2-picolyl)-benzamid

In eine auf -10°C abgekühlte Suspension von 4,0 g (0,0137 Mol) 2-Benzylselenobenzoesäure (aus Beispiel 1) in 20 ml Dichlormethan werden unter Rühren 1,8 g (0,0146 Mol) Chlormethylen-dimethyliminiumchlorid (Vilsmeier-Reagenz) eingetragen und 2 Stunden gerührt. Dann wird innerhalb von 10 Minuten eine Lösung von 1,49 g (0,0138 Mol) 2-Picolylamin und 3,0 g (0,03 Mol) Triethylamin in 10 ml Dichlormethan zugetropft und 14 Stunden gerührt. Anschließend wird nacheinander mit 50 ml Wasser, 50 ml 1 N NaOH, 50 ml Essigsäure (5%) und 50 ml Wasser ausgeschüttelt, die Dichlormethanphase getrocknet und eingeengt und der Rückstand 2 mal aus je 60 ml 2-Propanol umkristallisiert.
Ausbeute: 3,48 g (66,6% d.Th.)
Fp.: 141 - 142°C

### Beispiel 6

### 2-Benzylseleno-N-(3-picolyl)-benzamid

Analog Beispiel 5 aus:
(0,0137 Mol) 2-Benzylselenobenzoesäure (Beispiel 1)
1,49 g (0,0138 Mol) 3-Picolylamin
Ausbeute: 2,84 g (54,4% d.Th.) Fp.: 156 - 157°C

### Beispiel 7

### 2-Benzylseleno-N-(4-picolyl)-benzamid

Analog Beispiel 5 aus:
4,0 g (0,0137 Mol) 2-Benzylselenobenzoesäure (Beispiel 1)
1,49 g (0,0138 Mol) 4-Picolylamin
Ausbeute: 1,52 g (29,1% d.Th.)
Fp.: 151 - 152°C

### Beispiel 8

### 4,4-Dimethyl-2-(2-benzylseleno-3-fluorphenyl)-1,3-oxazolin

Zu einer auf -45° C abgekühlten Lösung von 30,0g 4,4-Dimethyl-2-(3-fluorphenyl)1,3-oxazolin in 400 ml getrocknetem Tetrahydrofuran werden 100ml einer 1,6 molaren Lösung von n-Butyllithium in n-Hexan so zugetropft, daß eine Temperatur von -40° C nicht überschritten wird (25 Minuten).

Nach zweistündigem Rühren bei -45° C wird das Reaktionsgemisch auf O° C erwärmt und anschließend eine Lösung von 54,7g (0,16Mol) Dibenzyldiselenid in 250ml getrocknetem Tetrahydrofuran innerhalb von 20 Minuten zugetropft. Das Reaktionsgemisch wird 18h bei Raumtemperatur gerührt. Der Kolbeninhalt wird in 300g Eis und 300ml Wasser aufgenommen und zweimal mit je 300ml Diethylether extrahiert. Die organische Phase wird nacheinander mit 200ml Wasser, 200ml 10%iger Natriumhydrogencarbonatlösung und 200ml Wasser ausgeschüttelt, über Natriumsulfat getrocknet und im Vakuum eingedampft. Das Rohprodukt (58,3g) wird durch Säulenchromatographie (Kieselgel/Dichlormethan) gereinigt.
Ausbeute: 43,5g (75% d. Th.)
Fp.: 95° C

### Beispiel 9

### 2-Benzylseleno-3-fluorbenzoesäure

Eine Suspension von 43,5g (0,12Mol) 4,4-Dimethyl-2-(2-benzylseleno-3-fluor-phenyl)-1,3-oxazolin in 102,6g (0,6 Mol) Benzylbromid wird bei Raumtemperatur 24 Stunden gerührt und die Lösung eingeengt. Der Rückstand wird in einem Gemisch aus 1,8l NaOH (20%ig) und 1,8l Methanol gelöst, 12 Stunden am Rückfluß erwärmt und der Kolbeninhalt mit 300ml Diethylether extrahiert. Die wäßrige Phase wird mit konz. HCl auf pH 1 eingestellt, der ausgefallenen Feststoff abgesaugt, mit 200 ml Wasser gewaschen und getrocknet. Der etherische Rückstand wird nach der Extraktion nochmals in Benyzlbromid gelöst und wie oben beschrieben behandelt. Nach Einstellung der wäßrigen Lösung auf pH 1 wird zuerst mit Wasser und nach der Trocknung mit n-Hexan nachgewaschen.
Gesamtausbeute: 29,9 g (80,6% d. Th.)
Fp.: 137-138° C

### Beispiel 10

### 2-Benzylseleno-3-fluor-N-(3-pyridyl)benzamid

Zu einer auf 0° C abgekühlten Lösung von 14,5 g (0,047 Mol) 2-Benzylseleno-3-fluor-benzoesäure und 6,54 ml Triethylamin in 90 ml Dichlormethan gibt man in fester Form und auf einmal 11,9 g (0,047 Mol) Bis-(2-oxo-3-oxazolidinyl)-phosphorylchlorid. Nach 10 Minuten Rühren werden 4,42 g (0,047 Mol) 3-Aminopyridin in 40 ml Dichlormethan bei Raumtemperatur zugetropft und nach weiteren 15 Minuten Rühren 6,54 ml Triethylamin in 20 ml Dichlormethan. Nach 48 h bei Raumtemperatur wird das Reaktionsgemisch mit 150 ml Wasser gewaschen und die organische Phase nacheinander mit 100 ml 2N NaOH, 100 ml 10%iger HCl und 100 ml Wasser ausgeschüttelt. Die organische Phase wird über Natriumsulfat getrocknet, im Wasserstrahlvakuum aufkonzentriert und über eine Säule (Kieselgel/Dichlormethan) filtriert. Die aufkonzentrierten sauberen Fraktionen werden dann in 350 ml eines Gemisches aus Dichlormethan/Hexan (8:2) umkristallisiert.
Ausbeute: 10,2 g (57% d. Th.)
Fp.: 130-131° C

## Patentansprüche

1. Benzylselenobenzamide der allgemeinen Formel I 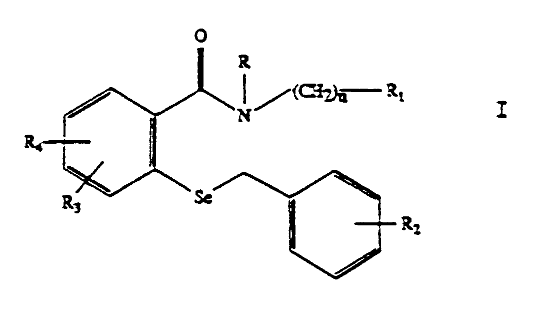 worin
R Wasserstoff, Methyl oder Ethyl bedeutet und
R₁ für die Pyridylgruppe steht, die gegebenenfalls durch Fluor, Chlor, Brom, C₁₋₄-Alkyl, C₁₋₄-Alkoxy, Hydroxy, Cyano, Amino, Dimethylamino oder Nitro substituiert sein kann und
R₂, R₃ und R₄ gleich oder verschieden sind und unabhängig voneinander für Wasserstoff, Fluor, Chlor, Brom, Trifluormethyl, C₁₋₄-Alkyl, C₁₋₄-Alkoxy, Hydroxy, Cyano oder Nitro stehen oder
R₃ ,R₄ zusammen für Methylendioxy stehen und
n Null oder 1 bedeutet.

2. Benzylselenobenzamide der allgemeinen Formel I,
Anspruch 1,
worin
n gleich Null ist und R und R₂ Wasserstoff bedeuten, während R₁ , R₃ und R₄ die in Anspruch 1 angegebene Bedeutung besitzen.

3. Benzylselenobenzamide der allgemeinen Formel I,
Anspruch 1,
worin
n gleich 1 ist und R und R₂ Wasserstoff bedeuten, während R₁ , R₃ und R₄ die in Anspruch 1 angegebene Bedeutung besitzen.

4. Verfahren zur Herstellung der Benzylselenobenzamide gemäß Formel I, dadurch gekennzeichnet, daß man in einem chlorierten Kohlenwasserstoff eine suspendierte Verbindung der Formel II 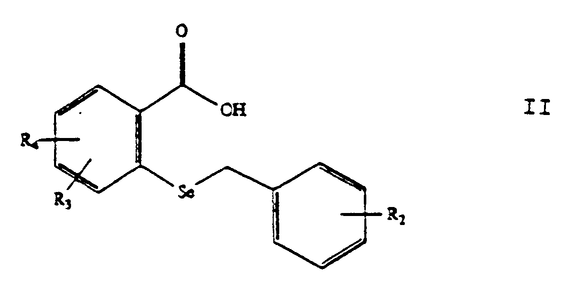 mit N,N'-Carbonyldiimidazol umsetzt, das Produkt dann mit einem Aminopyridin oder einem Picolylamin umsetzt und das entstandene Produkt aus dem Reaktionsgemisch abtrennt.

5. Verfahren zur Herstellung von Verbindungen nach Anspruch 1 bis 3, dadurch gekennzeichnet, daß man in einem chlorierten Kohlenwasserstoff eine Verbindung der allgemeinen Formel II mit 2-Chlor-1-methylpyridiniumjodid und einem Pyridin-haltigen Amin umsetzt und das Produkt aus dem Reaktionsgemisch abtrennt.

6. Verfahren zur Herstellung von Verbindungen nach Anspruch 1 bis 3, dadurch gekennzeichnet, daß man in einem chlorierten Kohlenwasserstoff eine Verbindung der allgemeinen Formel II mit Chlormethylendimethyliminiumchlorid und einem Pyridylamin oder Picolylamin umsetzt und das Produkt aus dem Reaktionsgemisch abtrennt.

7. Pharmazeutische Präparate, dadurch gekennzeichnet, daß sie entzündungshemmende Eigenschaften besitzen und als Wirkstoff eine Verbindung der Formel I gemäß den Ansprüchen 1 bis 3 im Gemisch mit üblichen pharmazeutischen Hilfs- und Trägerstoffen enthalten.

## Claims

1. Benzylselenobenzamides of the general formula I 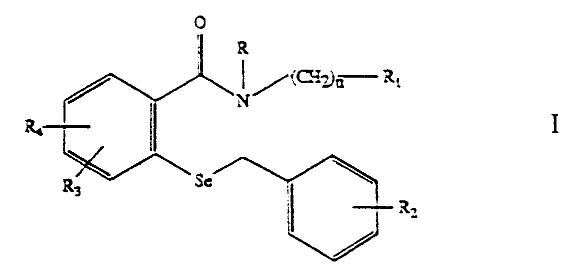 where
R is hydrogen, methyl or ethyl and
R₁ represents the pyridylgroup which can be substituted with fluorine, chlorine, bromine, C₁₋₄-alkyl, C₁₋₄-alkoxy, hydroxy, cyano, amino, dimethylamino or nitro and
R₂, R₃ and R₄ are identical or different and, taken separately, represent hydrogen, fluorine, chlorine, bromine, trifluoromethyl, C₁₋₄-alkyl, C₁₋₄-alkoxy, hydroxy, cyano or nitro or
R₃, R₄ taken together, represent methylenedioxy and
n is zero or 1.

2. The benzylselenobenzamides according to the general formula I, claim 1,
wherein
n equals zero and R and R₂ represent hydrogen, while R₁, R₃ and R₄ possess the meaning assigned in claim 1.

3. The benzylselenobenzamides according to the general formula I, claim 1,
wherein
n equals 1 and R and R₂ represent hydrogen; while R₁, R₃ and R₄ possess the meaning assigned in claim 1.

4. A method of preparation of the benzylselenobenzamides according to formula I, whereby a compound of formula II 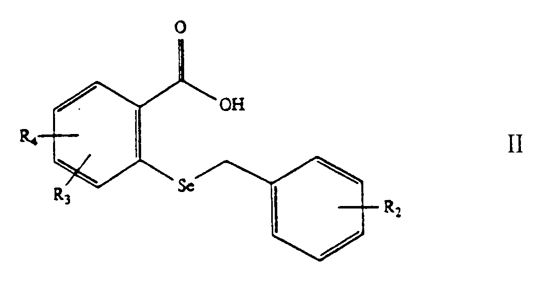 is suspended in a chlorinated hydrocarbon and reacted with N,N'- carbonyldiimidazole, the product is then reacted with an aminopyridine or a picolylamine and the product so formed is isolated from the reaction mixture.

5. A method of preparation of compounds according to claim 1 to 3, whereby a compound of general formula II is reacted in a chlorinated hydrocarbon with 2-chloro-1-methylpyridinium iodide and a pyridine-containing amine and the product so formed is isolated from the reaction mixture.

6. A method of preparation of compounds according to claim 1 to 3, whereby a compound of general formula II is reacted in a chlorinated hydrocarbon with chloromethylenedimethyliminium chloride and a pyridylamine or a picolylamine and the product so formed is isolated from the reaction mixture.

7. Pharmaceutical products having anti-inflammatory properties and comprising a compound of formula I according to claim 1 to 3 as an active ingredient in admixture with usual pharmaceutical additives and excipients.

## Revendications

1. Benzylsélénobenzamides de la formule générale I : 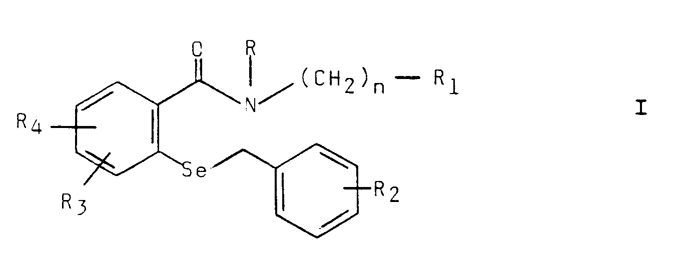 dans laquelle
R représente un atome d'hydrogène, le radical méthyle ou éthyle, et
R₁ représente le radical pyridyle, qui peut éventuellement être substitué par du fluor, du chlore, du brome, des radicaux alkyle en C₁-C₄, alcoxy en C₁-C₄, hydroxyle, cyano, amino, diméthylamino ou nitro, et
R₂, R₃ et R₄ ont des significations identiques ou différentes et représentent, chacun indépendamment l'un de l'autre, un atome d'hydrogène, de fluor, de chlore, de brome, le radical trifluorométhyle, un radical alkyle en C₁-C₄, alcoxy en C₁-C₄, hydroxyle, cyano ou nitro, ou bien
R₃, R₄ forment ensemble un radical méthylènedioxy, et
n est égal à zéro ou à 1.

2. Benzylsélénobenzamides de la formule générale I, selon la revendication 1,
caractérisés en ce que
n est égal à zéro et R et R₂ représentent chacun un atome d'hydrogène, cependant que R₁, R₃ et R₄ ont les significations qui leur ont été attribuées dans la revendication 1.

3. Benzylsélénobenzamides de la formule générale I, selon la revendication 1,
caractérisés en ce que
n est égal à 1 et R et R₂ représentent chacun un atome d'hydrogène, cependant que R₁, R₃ et R₄ ont les significations qui leur ont été attribuées dans la revendication 1.

4. Procédé de préparation de benzylsélénobenzamides répondant à la formule générale I, caractérisé en ce que, dans un hydrocarbure chloré, on fait réagir un composé en suspension de la formule II : 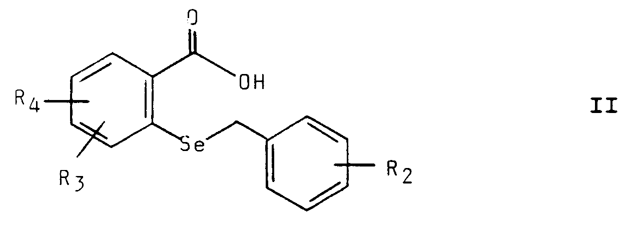 avec le N,N'-carbonyldiimidazole, on fait ensuite réagir le produit avec une aminopyridine ou une picolylamine et on sépare le produit formé du mélange de réaction.

5. Procédé de préparation de composés selon l'une quelconque des revendications 1 à 3, caractérisé en ce que, dans un hydrocarbure chloré, on fait réagir un composé de la formule générale II avec l'iodure de 2-chloro-1-méthylpyridinium et une amine contenant de la pyridine et on sépare le produit du mélange de réaction.

6. Procédé de préparation de composés selon l'une quelconque des revendications 1 à 3, caractérisé en ce que, dans un hydrocarbure chloré, on fait réagir un composé de la formule générale II avec le chlorure de chlorométhylènediméthyliminium et une pyridylamine ou une picolylamine et on sépare le produit du mélange de réaction.

7. Préparations pharmaceutiques, caractérisées en ce qu'elles possèdent des propriétés anti-inflammatoires et contiennent, à titre de principe actif, un composé de la formule I selon l'une quelconque des revendications 1 à 3 en mélange à des véhicules ou excipients pharmaceutiques usuels.
